# EUROPÄISCHE PATENTANMELDUNG

(11) **EP 2 742 920 A1**
(43) Veröffentlichungstag der Anmeldung: **18.06.2014**
(21) Anmeldenummer: 13186474.6
(22) Anmeldetag: 27.09.2013
(51) Int. Cl.: A61F 5/56

(54) **Fluidgefüllter Mundeinsatz sowie Verwendung eines derartigen Mundeinsatzes als Sportschiene und als Beißkissen**

(30) Priorität: 14.12.2012 DE 102012223146
(71) Anmelder: Ingenieurbüro Laich, 91080 Uttenreuth (DE)
(72) Erfinder: Laich, Hans, 91080 Uttenreuth (DE)
(74) Vertreter: Rau, Schneck & Hübner Patentanwälte Rechtsanwälte PartGmbB

(57) **Zusammenfassung**

Ein fluidgefüllter Mundeinsatz umfasst zwei Aufbisskissen (2) und mindestens einen die Aufbisskissen (2) verbindenden Verbindungsschlauch (6, 7) zum Austausch von Fluid zwischen den Aufbisskissen (2), wobei eine die Aufbisskissen (2) verbindende Haltestruktur für einen sicheren Halt des Mundeinsatzes (1) in einem Mund vorgesehen ist, und wobei der mindestens eine Verbindungsschlauch (6, 7; 6, 7; 19) die Haltestruktur bildet.

## Beschreibung

Die Erfindung betrifft einen fluidgefüllten Mundeinsatz sowie dessen Verwendung als Sportschiene und als Beißkissen.

Aus dem Stand der Technik sind fluidgefüllte Mundeinsätze beispielsweise als hydraulische Schienen zur Behandlung der Kiefergelenke bekannt. Eine derartige Schiene zur Behandlung der craniomandibulären Dysfunktion (CMD) ist aus der DE 102 39 464 B4 bekannt. Die Schiene weist zwei mit Flüssigkeit gefüllte Kissen auf, die mittels eines Schlauchs zum Flüssigkeitsaustausch miteinander verbunden sind. Um die Schiene im Mund zu halten, sind metallische Klammern erforderlich. Darüber hinaus sind an der Schiene jeweils im Bereich der Kissen ein bukkaler und ein lingualer Schenkel zur Positionierung der Schiene im Mund erforderlich. Der Halt einer derartigen Schiene erfolgt insbesondere durch eine von einem Zahnarzt anzubringende Silikonunterfütterung im Bereich der Kissen. Das Einsetzen einer derartigen Schiene ist aufwändig und insbesondere für einen Patienten ohne ärztliche Anpassung nicht möglich.

Die US 3 532 091 offenbart einen Mundeinsatz als Sportschiene. Ein derartiger Mundeinsatz weist mehrere Aufbisskissen auf, die durch einen Verbindungsschlauch miteinander in Fluidverbindung stehen. Die Aufbisskissen sind im Wesentlichen L-förmig ausgeführt, um den Halt der Sportschiene im Mund zu verbessern. Ein sicherer Halt des Mundeinsatzes im Mund ist nicht gewährleistet.

Aus der DE 29 41 463 A1 ist eine fluidgefüllte Schiene bekannt. Bei dieser Schiene sind keine Halteelemente vorgesehen. Der sichere Halt der Schiene im Mund ist nicht gewährleistet. Insbesondere ist es möglich, dass die Schiene unbeabsichtigt, insbesondere während des Schlafens des Patienten, aus dem Mund herausfallen kann.

Es ist eine Aufgabe der vorliegenden Erfindung, einen fluidgefüllten Mundeinsatz derart zu verbessern, dass er unkompliziert einsetzbar ist und insbesondere sicher im Mund eines Patienten anbringbar ist.

Diese Aufgabe ist durch einen fluidgefüllten Mundeinsatz mit den Merkmalen des Anspruchs 1 gelöst. Erfindungsgemäß wurde erkannt, dass eine zwei Aufbisskissen verbindende Haltestruktur einen sicheren Halt eines Mundeinsatzes in einem Mund eines Patienten gewährleistet. Die Haltestruktur ist insbesondere derart ausgeführt, dass sie in einem Tragezustand eines Patienten in einem vorderen Bereich des Gebisses, insbesondere im Bereich der Schneidezähne, angeordnet ist. Insbesondere ist die Haltestruktur unauffällig ausgeführt. Die beiden Aufbisskissen sind durch mindestens einen Verbindungsschlauch und insbesondere durch mindestens zwei Verbindungsschläuche zum Austausch von Fluid zwischen Aufbisskissen, die insbesondere zwischen den linken Backenzähnen bzw. zwischen den rechten Backenzähnen eines Gebisses anzuordnen sind, verbunden. Die Haltestruktur ist durch den mindestens einen Verbindungsschlauch gebildet. Die Haltestruktur ist insbesondere durch genau einen Verbindungsschlauch gebildet. Die Haltestruktur wird insbesondere dadurch gebildet, dass der mindestens eine Verbindungsschlauch aus einem Schlauch-Rohmaterial hergestellt ist. Dadurch ist es nicht erforderlich, einzelne Folien miteinander zu verbinden. Der verwendete Schlauch weist eine Eigenstabilität und Eigensteifigkeit auf, die die Ausbildung der Haltestruktur unterstützen. Die Handhabung des Mundeinsatzes ist vereinfacht. Aufgrund der gebildeten Haltestruktur, die insbesondere rahmenartig ausgeführt sein kann, weist der Mundeinsatz insgesamt eine verbesserte Formstabilität auf. Es ist insbesondere nicht erforderlich, zusätzliche Halteelemente in Form metallischer Klammern und/oder bukkaler oder lingualer Schenkel zum Anliegen an den Zähnen vorzusehen. Der Mundeinsatz ist unkompliziert ausgeführt und weist insbesondere keine als störend empfundene Halteelemente auf. Der Tragekomfort des Mundeinsatzes ist verbessert. Insbesondere ist eine ärztliche Anpassung des Mundeinsatzes beim Einsetzen in den Mund eines Patienten nicht erforderlich. Ein Patient kann den erfindungsgemäßen Mundeinsatz selbstständig einsetzen und wieder herausnehmen. Insbesondere sind genau zwei Aufbisskissen vorgesehen. Es ist auch möglich, mehr als zwei Aufbisskissen in dem erfindungsgemäßen Mundeinsatz zu verwenden. Es ist beispielsweise denkbar, jeweils zwei Aufbisskissen paarweise im Bereich der Backenzähne vorzusehen, sodass insgesamt ein Mundeinsatz vier Aufbisskissen aufweisen kann. Die paarweise angeordneten Kissen können beispielsweise L-förmig angeordnet sein und durch mindestens einen Verbindungsschlauch miteinander verbunden sein. Ein Schenkel des L kann zwischen den Backenzähnen von Oberkiefer und Unterkiefer angeordnet sein. Der andere Schenkel des L kann an einer Innenseite der Backenzähne, also zwischen Zunge und Backenzähnen, angeordnet sein.

Ein derartiger Mundeinsatz kann beispielsweise als zur Diagnose und zur Behandlung der CMD verwendet werden. Weiterhin ist eine Verwendung als Beißkissen denkbar. Ein derartiger Mundeinsatz ermöglicht Schutz vor Wangenbeißen und/oder Zungenbeißen beispielsweise bei Epilepsie oder Parafunktionen. Darüber hinaus kann ein derartiger Mundeinsatz die Zähne und/oder die Kiefergelenke vor Verletzungen durch Stoßeinwirkung auf den Unterkiefer schützen. Insbesondere sind zusätzliche Schutzelemente nicht zwingend erforderlich.

Ein Mundeinsatz gemäß Anspruch 2 ist kostengünstig herstellbar. Insbesondere ist ein verwendeter Kunststoff weich und biologisch unbedenklich. Ein derartiger Kunststoff ist beispielsweise Ethylenvinylacetat (EVA) oder Silikon. Ein derartiger Kunststoff ist geeignet, über einen längeren Zeitraum im Mundraum einer Person zu verbleiben. Insbesondere ist ein derartiger Kunststoff transparent. Der die Hülle bildende Kunststoff kann einen Wirkstoff, einen Geschmacksstoff und/oder einen Geruchsstoff enthalten, die je nach Konzentration der Stoffe kontinuierlich mit einer vorgegebenen Rate in den Mundraum des Patienten abgegeben werden. Zusätzlich oder alternativ ist es auch möglich, dass das Fluid einen Wirkstoff, einen Geschmacksstoff und/oder einen Geruchsstoff aufweist. Diese Stoffe können durch die Hülle des Mundeinsatzes diffundieren und an den Mundraum des Patienten, insbesondere in homöopathischen Dosen, dauerhaft an den Patienten abgegeben werden. Die Herstellung der Kunststoffhülle erfolgt insbesondere durch Blasformen oder durch Rotationsformen. Aufgrund der einteiligen Herstellung einer Hülle des Mundeinsatzes ist dieser im Wesentlichen nahtlos ausgeführt. Das bedeutet, dass insbesondere im Vergleich zu einer umlaufenden Naht, die beim Verschweißen einzelner Folienlagen miteinander resultiert, seitlich überstehende, störende Überstandsabschnitte bei dem Mundeinsatz vermieden sind. Eine infolge eines Herstellungsprozesses verbleibende Naht nimmt ein Patient vielmehr als nicht störende Auswölbung in der Außenkontur des Mundeinsatzes wahr. Diese Auswölbung ist nicht störend. Dadurch ist der Tragekomfort für einen Patienten zusätzlich verbessert.

Ein Mundeinsatz nach Anspruch 3 weist eine erhöhte Formstabilität auf. Es sind genau zwei Verbindungsschläuche vorgesehen, die die Aufbisskissen zum Fluidaustausch miteinander verbinden. Die Verbindungsschläuche bilden eine Haltestruktur derart, dass ein sicherer Halt des Mundeinsatzes in dem Mund gewährleistet ist. Das bedeutet, dass die Verbindungsschläuche zwei Funktionen erfüllen, nämlich den Fluidaustausch zwischen den Aufbisskissen einerseits und den verbesserten, sicheren Halt des Mundeinsatzes im Mund andererseits. Die von den Verbindungsschläuchen gebildete Haltestruktur weist eine Haltestrukturfläche auf. Die Haltestrukturfläche ist insbesondere gekrümmt und entspricht insbesondere einer von den Zahnreihen vorgegebenen Kontur. Die Haltestruktur ist quer und insbesondere senkrecht zu einer Aufbissebene orientiert. Die Aufbissebene wird durch die Aufbisskissen, die im Wesentlichen flächig ausgebildet sind, festgelegt. Flächig bedeutet in diesem Zusammenhang, dass eine Ausdehnung des Kissens parallel zur Kaufläche der Backenzähne, also eine Länge und eine Breite des Aufbisskissens, deutlich größer sind als eine Dicke des Aufbisskissens. Die Dicke des Aufbisskissens ist senkrecht zur Kaufläche, also senkrecht zur Aufbissebene, orientiert. Insbesondere beträgt die Dicke des Aufbisskissens zwischen 2 mm und 6 mm und insbesondere 4 mm. Die Breite des Aufbisskissens beträgt etwa zwischen 11,25 mm und 18,75 mm und insbesondere 15 mm. Die Länge des Aufbisskissens beträgt etwa zwischen 20 mm und 30 mm und insbesondere 25 mm. Die Auffbisskissen sind insbesondere auf den 5er- und 6er-Zähnen eines menschlichen Gebisses angeordnet. Je nach Anwendungsfall können die Aufbisskissen sich auch zumindest teilweise auf die 4er-Zähne und/oder auf die 7er-Zähne des menschlichen Gebisses erstrecken. Die Aufbissebene ist insbesondere parallel zu einem Aufbissspalt des Gebisses orientiert. Der Aufbissspalt ist der Spalt zwischen Zähnen des Oberkiefers und des Unterkiefers, der dadurch resultiert, dass die Aufbisskissen zwischen den Zahnreihen des Oberkiefers und des Unterkiefers angeordnet sind.

Ein Mundeinsatz gemäß Anspruch 4 ermöglicht eine besonders vorteilhafte Integration der Haltestruktur im Mund des Patienten. Ein oberer Verbindungsschlauch ist derart zwischen den beiden Aufbisskissen angeordnet, dass er oberhalb der Schneidezähne des Oberkiefers in einem Tragezustand des Mundeinsatzes angeordnet ist. Das bedeutet, dass der obere Verbindungsschlauch im Tragezustand labial an den Schneidezähnen vorbeigeführt ist. Entsprechend ist ein unterer Verbindungsschlauch unterhalb der Schneidezähne des Unterkiefers anordenbar. Der obere Verbindungsschlauch und der untere Verbindungsschlauch bilden insbesondere einen Rahmen, der eine verbesserte Formstabilität aufweist. Die Verbindungsschläuche sind unauffällig und damit insbesondere optisch nicht störend im Mund des Patienten anordenbar. Die Verbindungsschläuche verhindern ein beabsichtigtes Lösen des Mundeinsatzes aus dem Mund. Zusätzliche Halteelemente sind entbehrlich.

Ein Mundeinsatz nach Anspruch 5 ermöglicht einen verbesserten Tragekomfort. Aufgrund eines insbesondere konvex geformten Konturelements der Verbindungsschläuche im Bereich der Schlauchmitte, also in einem mittigen Bereich zwischen den beiden Aufbisskissen, ist ein möglicherweise als störend empfundener Kontakt des oberen bzw. unteren Lippenbändchens mit dem Mundeinsatz verhindert. Gleichzeitig wirkt das jeweilige Konturelement im Bereich der Schlauchmitte der Verbindungsschläuche ähnlich einer Sicke und führt zu einer zusätzlichen Formstabilität des Mundeinsatzes.

Ein Mundeinsatz gemäß Anspruch 6 ermöglicht eine zusätzliche Schutzfunktion der Zähne, der Kiefergelenke, der Lippeninnenseiten und/oder der Wangen eines Patienten. Ein derartiger Mundeinsatz ermöglicht Schutz vor Stoß- und/oder Schlageinwirkungen auf den Mund und auf den Unterkiefer. Ein derartiger Mundeinsatz kann insbesondere als Sportschiene verwendet werden.

Ein Mundeinsatz nach Anspruch 7 umfasst mindestens zwei und insbesondere genau drei Verbindungsschläuche. Die von den Verbindungsschläuchen gebildete Haltestruktur weist eine Haltestrukturfläche auf, die im Wesentlichen parallel zu einer von den Aufbisskissen festgelegten Aufbissebene orientiert ist. Insbesondere ist die Haltestrukturfläche parallel zu der Aufbissebene orientiert. Ein derartiger Mundeinsatz ermöglicht einen sicheren Halt im Mund eines Patienten. Ein derartiger Mundeinsatz kann beispielsweise als Beißkissen verwendet werden.

Ein Mundeinsatz nach Anspruch 8 ermöglicht eine intuitive Anpassung der Position des Mundeinsatzes im Mund durch den Patienten selbst. Dadurch, dass die Verbindungsschläuche in der Aufbissebene beabstandet zueinander und insbesondere parallel zueinander angeordnet sind, wird jeweils zwischen zwei benachbarten Verbindungsschläuchen ein Zwischenraum gebildet. Der Patient kann mit den Schneidezähnen des Ober- bzw. des Unterkiefers in die Zwischenräume eingreifen und somit den Mundeinsatz relativ zu den Kiefern bzw. zu den Zähnen positionieren.

Ein Mundeinsatz nach Anspruch 9 weist einen erhöhten Tragekomfort auf. Dadurch, dass die Verbindungsschläuche in einer gemeinsamen Hülle integriert sind, nimmt der Patient den Mundeinsatz als einteiliges, im Wesentlichen U-förmiges, flächiges Kissen wahr. Im Bereich der Backenzähne, also an den Enden des U, sind die Aufbisskissen positioniert. Im Bereich des Bogens des U sind die in der Hülle integrierten Verbindungsschläuche angeordnet. Die Hülle ist insbesondere derart weich, dass ein Patient die Verbindungsschläuche mit den Zähnen ertasten und in die Zwischenräume eingreifen kann.

Ein Mundeinsatz nach Anspruch 10 ermöglicht eine zusätzliche Funktionalität. Mittels einer Kaukraftmesseinheit kann eine Kaukraft bzw. ein Kaudruck gemessen werden. Dazu weist eine Kaukraftmesseinheit ein 2-Wege-Ventil und einen Druckgeber auf. Derart gemessene Kaukraftwerte können zur Diagnose von fehlerhaftem Kauverhalten eingesetzt werden.

Ein Mundeinsatz nach Anspruch 11 ermöglicht eine Kaukraftmessung mit einer verbesserten Genauigkeit. Dadurch, dass die Kaukraftmesseinheit in einem der Verbindungsschläuche integriert ist, kann ein Fluiddurchfluss definiert ermittelt werden. Insbesondere weisen die Verbindungsschläuche einen gleichbleibenden Innendurchmesser auf, der einen Strömungsquerschnitt definiert. Es ist auch möglich, mehrere Kaukraftmesseinheiten innerhalb eines Verbindungsschlauchs und/oder in mehreren Verbindungsschläuchen vorzusehen. Es ist möglich, Kaukraftmesswerte verschiedener Kaukraftmesseinheiten miteinander abzugleichen. Dadurch ist die Messgenauigkeit zusätzlich erhöht.

Ein Mundeinsatz nach Anspruch 12 ermöglicht eine vorteilhafte Herstellung des Mundeinsatzes und insbesondere eine integrierte Ausbildung der Haltestruktur.

Ein Mundeinsatz gemäß Anspruch 13 weist einen dickwandigen Schlauch als mindestens einen Verbindungsschlauch auf. Als dickwandig wird der Schlauch mit einer Wandstärke im Bereich von 0,5 mm bis 1,0 mm bezeichnet. Insbesondere beträgt die Wandstärke des Schlauchs 0,6 mm bis 0,8 mm und insbesondere etwa 0,7 mm. Bezogen auf den Außendurchmesser des Verbindungsschlauchs beträgt die Wandstärke mindestens 15 %, insbesondere mindestens 20 % und insbesondere mindestens 25 %. Insbesondere ist die Ausführung des mindestens einen Verbindungsschlauchs als dickwandiger Schlauch gegenüber Mundeinsätzen aus dünnwandigen Folien, die beispielsweise aus PA 66 hergestellt sind, vorteilhaft. Derart aus Folien hergestellte Mundeinsätze haben keine oder keine ausreichende Eigenstabilität. Derartige Mundeinsätze benötigen eine zusätzliche Haltestruktur, die beispielsweise bei der US 3 532 091 durch sattelförmige, labiale und/oder bukkale Kissen realisiert ist. Alternativ kann eine zusätzliche Haltestruktur auch durch einen in dem mindestens einen Verbindungsschlauch angeordneten Metalldraht ausgeführt sein. Derartige Mundeinsätze sind komplex ausgeführt. Der Tragekomfort kann insbesondere durch die lingualen und/oder bukkalen Kissen beeinträchtigt sein.

Die Verwendung eines Mundeinsatzes als Sportschiene gemäß Anspruch 14 ermöglicht eine besonders vorteilhafte Anwendung bei Sportlern. Derartige Sportschienen dienen einerseits dem Schutz der Zähne und der Kiefergelenke vor Schlagverletzungen. Andererseits dienen derartige Sportschienen auch zum Schutz der Lippeninnenseiten vor Verletzungen an festen Zahnspangen. Erfindungsgemäß wurde nun erkannt, dass in Folge eines natürlichen Reflexes ein Sportler unter Stress und Druck, der beispielsweise in einer Wettkampfsituation auftreten kann, die Zähne zusammenpresst. Dadurch kann die Kaumuskulatur verkrampfen. Der Körper produziert das Stresshormon Cortisol, wodurch die Konzentration geschwächt wird und Ermüdung eintritt. Durch die Verwendung des erfindungsgemäßen Mundeinsatzes mit fluidgefüllten Aufbisskissen, die einen Fluidaustausch untereinander ermöglichen, ist eine Druckausgleichsfunktion zwischen dem rechten und dem linken Aufbisskissen ermöglicht. Dadurch kann das Verkrampfen der Kaumuskulatur gehemmt und insbesondere verhindert werden. Verkrampfungen entstehen nicht bzw. können gelöst werden. Das Risiko einer Konzentrationsschwächung oder einer Ermüdung ist reduziert. Weiterhin ist das Risiko, dass zahnschädigende Spitzenbelastungen in Folge des verkrampften Zubeißens auftreten können, reduziert.

Die Verwendung eines Mundeinsatzes als Beißkissen gemäß Anspruch 15 ermöglicht eine besonders vorteilhafte Spontanrelaxierung der Kaumuskulatur und die Entlastung der Kiefergelenke. Weiterhin ist eine neuromuskuläre Deprogrammierung des stomatognathen Systems verbessert, insbesondere durch Harmonisierung der Kaumuskulatur und der Unterkieferposition. Darüber hinaus erhält der Patient insbesondere die Möglichkeit, das Beißkissen intuitiv mittels der Schneidezähne im Mund vorteilhaft zu positionieren.

Ausführungsbeispiele der Erfindung werden nachfolgend anhand der Zeichnung näher erläutert. In dieser zeigen:
- Fig. 1: eine perspektivische schematische Darstellung eines erfindungsgemäßen Mundeinsatzes mit zwei Verbindungsschläuchen in einem Mund eines Patienten zur Verwendung als Beißkissen,
- Fig. 2: eine Fig. 1 entsprechende Darstellung des Mundeinsatzes mit zusätzlichen Schutzelementen gemäß einer weiteren Ausführungsform zur Verwendung als Sportschiene,
- Fig. 3: eine Draufsicht auf einen Mundeinsatz gemäß einer weiteren Ausführungsform zur Verwendung als Beißkissen, und
- Fig. 4: eine Schnittdarstellung gemäß Linie IV-IV in Fig. 3.

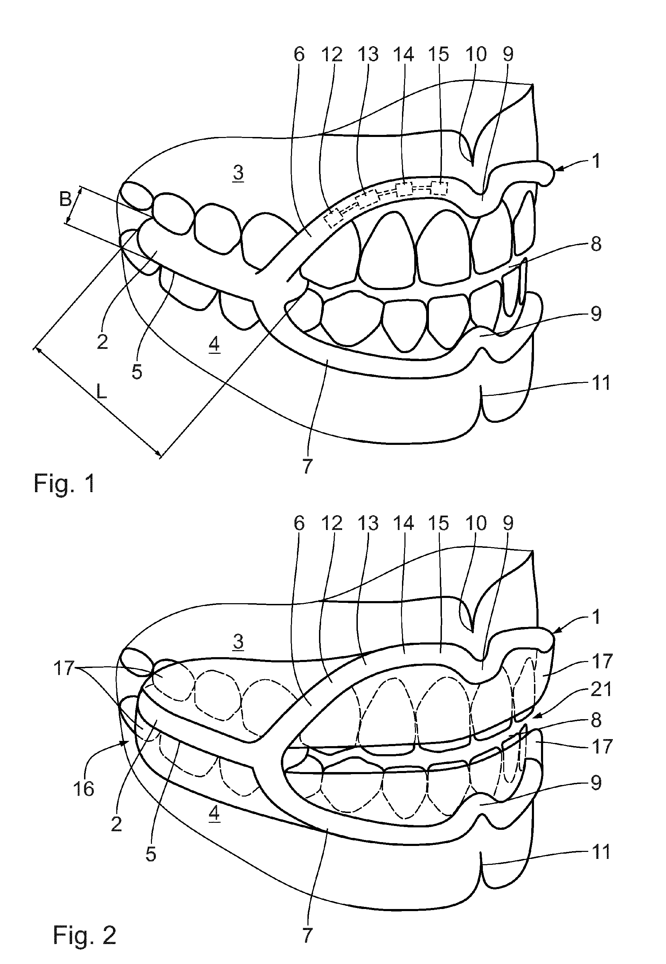

Ein in Fig. 1 dargestellter Mundeinsatz 1 weist zwei im Wesentlichen identische Aufbisskissen 2 auf. Der Mundeinsatz 1 weist insgesamt eine U-förmige Struktur auf. Die Form des Mundeinsatzes 1 entspricht im Wesentlichen der Form der Zahnreihen des menschlichen Gebisses. An den jeweiligen Enden des U sind die beiden Aufbisskissen 2 angeordnet. Aufgrund der perspektivischen Darstellung in Fig. 1 ist ausschließlich das zwischen den rechten Backenzähnen des Oberkiefers 3 und des Unterkiefers 4 angeordnete Aufbisskissen 2 erkennbar. Die Aufbisskissen 2 sind, so wie der Mundeinsatz 1 insgesamt, mit einem Fluid gefüllt. Das Fluid ist insbesondere Glycerin. Es sind auch andere Flüssigkeiten wie beispielsweise Wasser oder Öl zur Verwendung in dem Mundeinsatz 1 denkbar. Es ist auch möglich, gelartige Substanzen als Fluid in dem Mundeinsatz 1 zu verwenden. Es ist auch möglich, den Mundeinsatz 1 mit einem gasförmigen Fluid zu füllen.

Die Aufbisskissen 2 weisen eine äußere Kunststoffhülle 5 auf. Die Kunststoffhülle 5 begrenzt das Aufbisskissen 2 und umgibt einen Innenraum zur Bevorratung des Fluids. Das Aufbisskissen 2 hat im Wesentlichen eine flächenhafte Geometrie. Das bedeutet, dass das Aufbisskissen 2 eine Länge L entlang der Zahnreihe und parallel zu einer Kaufläche der Backenzähne angeordnet ist. Die Länge L und die Breite B legen die im Wesentlichen rechteckförmige Fläche des Aufbisskissens 2 parallel zur Kaufläche der Zähne fest. Das Aufbisskissen 2 weist weiterhin eine Dicke auf, die senkrecht zur Kaufläche orientiert ist.

Die Kunststoffhülle 5 und der Innendruck des Fluids in dem Mundeinsatz 1 ermöglichen, dass der Mundeinsatz 1 flexibel ist. Das bedeutet, dass in Folge eines Aufbeißens des Patienten auf mindestens eines der Aufbisskissen 2 Fluid verdrängt werden kann.

Die beiden Aufbisskissen 2 sind durch einen oberen Verbindungsschlauch 6 und einen unteren Verbindungsschlauch 7 miteinander in Fluidverbindung. Das bedeutet, dass ein Austausch von Fluid zwischen den Aufbisskissen 2 durch die Verbindungsschläuche 6, 7 möglich ist. Es ist auch möglich, dass genau ein Verbindungsschlauch vorgesehen ist.

Die flächenhaften Aufbisskissen 2 legen eine Aufbissebene fest, die parallel zu den flächigen Aufbisskissen orientiert ist. Die Aufbissebene ist zwischen dem Oberkiefer 3 und dem Unterkiefer 4, insbesondere parallel zu den Zahnreihen angeordnet. Die Aufbissebene ist in einem zwischen dem Oberkiefer 3 und dem Unterkiefer 4 angeordneten Aufbissspalt 8 angeordnet. Der Aufbissspalt 8 resultiert aus dem Abstand des Oberkiefers 3 zu dem Unterkiefer 4 in Folge des Aufbisskissens 2.

Die Verbindungsschläuche 6, 7 bilden eine rahmenförmige Haltestruktur, die eine Haltestrukturfläche vorgibt. Die Haltestrukturfläche ist gekrümmt und entspricht einer Frontfläche der Mundpartie des Patienten. Insbesondere ist die Haltestruktur quer und insbesondere senkrecht zu der Aufbissebene orientiert. Der obere Verbindungsschlauch 6 ist in der Trageposition gemäß Fig. 1 im Grenzbereich zwischen Zahnfleisch und dem sichtbaren Teil der Schneidezähne des Oberkiefers 3 angeordnet. Entsprechend ist der untere Verbindungsschlauch 7 im Grenzbereich zwischen Zahnfleisch und dem sichtbaren Teil der Schneidezähne des Unterkiefers 4 in der Trageposition angeordnet. Als nicht-sichtbarer Teil eines Zahns wird der von dem Zahnfleisch verdeckte und/oder in einem Kieferknochen angeordnete Teil eines Zahns verstanden. Jeweils im Bereich der Schlauchmitte weisen der obere Verbindungsschlauch 6 und der untere Verbindungsschlauch 7 ein konvex geformtes Konturelement 9 auf. Dadurch ist gewährleistet, dass das obere Lippenbändchen 10 bzw. das untere Lippenbändchen 11 durch das Tragen des Mundeinsatzes 1 nicht beeinträchtigt werden.

Der Mundeinsatz 1 weist weiterhin eine Kaukraftmesseinheit auf, die ein 2-Wege-Ventil 12 und einen damit verbundenen Druckgeber 13 aufweist. Das 2-Wege-Ventil 12 und der Druckgeber 13 sind in Fig. 1 schematisch dargestellt. Das 2-Wege-Ventil 12 ermöglicht eine Durchflusssteuerung. Der Druckgeber 13 ermöglicht die Erfassung des Drucks des Fluids, das durch das 2-Wege-Ventil 12 strömt. In Abhängigkeit der Strömungsrichtung, also entweder von dem linken Aufbisskissen 2 zu dem rechten Aufbisskissen 2 oder umgekehrt sowie in Abhängigkeit des gemessenen Drucks, kann eine Kaukraft sowohl qualitativ als auch quantitativ erfasst werden.

Die Kaukraftmesseinheit, also das 2-Wege-Ventil 12 und der Druckgeber 13, sind in dem oberen Verbindungsschlauch 6 integriert. Der Patient beißt nicht auf den oberen Verbindungsschlauch 6. Die Kaukraftmesseinheit wird durch das Aufbeißen des Patienten nicht beeinträchtigt. Gleichzeitig ist eine Irritation des Patienten durch ein Aufbeißen auf die Kaukraftmesseinheit ausgeschlossen. Es ist auch möglich, die Kaukraftmesseinheit in dem unteren Verbindungsschlauch 7 anzuordnen. Es ist auch möglich, mehrere Kaukraftmesseinheiten hintereinander, also entlang der Strömungsrichtung von dem einen zu dem anderen Aufbisskissen 2 in einem Verbindungsschlauch in Reihe, oder in mehreren Kaukraftmesseinheiten parallel, also in dem oberen Verbindungsschlauch 6 und in dem unteren Verbindungsschlauch 7 anzuordnen. Es ist auch möglich, die Kaukraftmesseinheit parallel und in Reihe zueinander anzuordnen. Aufgrund der Anbringung mehrerer Kaukraftmesseinheiten in den Verbindungsschläuchen 6, 7 ist die Messegenauigkeit verbessert.

Die Kaukraftmesseinheit kann ein Datenübertragungselement 14 und ein Speicherelement 15 aufweisen. Das Datenübertragungselement 14 dient zur Signalübertragung gemessener Kauwerte an eine externe Auswerteeinheit. Mittels der externen Auswerteeinheit können die übertragenen Kauwerte für eine weitere Diagnose oder Analyse des Kauverhaltens eines Patienten untersucht werden. Insbesondere ermöglicht das Datenübertragungselement 14 eine drahtlose Signalübertragung. Das Speicherelement 15 ermöglicht ein lokales Speichern gemessener Kaukraftwerte. Die gespeicherten Kaukraftwerte können zu einem späteren Zeitpunkt ausgelesen und an eine Auswerteeinheit übertragen werden. Dadurch ist es möglich, ein über einen längeren Zeitraum erstelltes Kaukraftwert-Profil zu ermitteln. Das Speicherelement 15 kann entnehmbar, also von dem Mundeinsatz 1 trennbar, ausgeführt sein, um das Auslesen der Daten zu vereinfachen. Es ist auch möglich, dass das Speicherelement 15 eine Standardschnittstelle und insbesondere einen Stecker oder eine Buchse wie Mini-USB aufweist.

Fig. 2 zeigt eine weitere Ausführung eines Mundeinsatzes 16. Komponenten, die denjenigen entsprechen, die vorstehend unter Bezugnahme auf die Fig. 1 bereits erläutert wurden, tragen die gleichen Bezugsziffern und werden nicht nochmals im Einzelnen diskutiert.

Der Mundeinsatz 16 gemäß Fig. 2 dient insbesondere zur Verwendung als Sportschiene. Dazu weist der Mundeinsatz 16 angeformte Schutzelemente 17 auf. Die Schutzelemente 17 sind beispielsweise an einem der Aufbisskissen 2 und dem oberen Verbindungsschlauch 6 bzw. dem unteren Verbindungsschlauch 7 angeformt. In einem vorderen Bereich zum Anliegen an den Schneidezähnen sind die Schutzelemente 17 jeweils nur an dem oberen Verbindungsschlauch 6 oder an dem unteren Verbindungsschlauch 7 angeformt. Die Schutzelemente 17 im Bereich der Aufbisskissen 2 sind derart angeformt, dass der Mundeinsatz 16 in diesem Bereich eine geschlossene, schützende Oberfläche bildet. Im Bereich der Schneidezähne sind die Schutzelemente 17 bezüglich der Aufbissebene beabstandet zueinander angeordnet, so dass der Mundeinsatz 16 eine spaltförmige Öffnung als Mundspalt 21 entsprechend dem Aufbissspalt 8 aufweist. Dadurch ist gewährleistet, dass ein den Mundeinsatz 16 tragender Sportler einerseits geschützt ist und andererseits atmen und/oder Flüssigkeit aufnehmen kann. Es ist auch möglich, in dem vorderen Bereich zum Anliegen an den Schneidezähnen ein einteiliges Schutzelement 17 vorzusehen, das an dem oberen Verbindungsschlauch 6 und an dem unteren Verbindungsschlauch 7 befestigt ist. Das einteilige Schutzelement 17 weist dann einen entsprechenden Mundspalt 21 auf. Durch das einteilige Schutzelement 17 ist die Formstabilität des Mundeinsatzes 16 insgesamt verbessert. Die Schutzelemente 17 sind insbesondere aus einem harten Kunststoff hergestellt. Sie dienen dem Schutz der Zähne, der Kiefergelenke, der Lippeninnenseiten und der Wangen. Dadurch, dass der Mundeinsatz 16 einen Fluidfluss zwischen den Aufbisskissen 2 ermöglicht, steht eine Sportschiene mit Druckausgleichsfunktion zwischen dem rechten und dem linken Aufbisskissen 2 zur Verfügung.

Es ist auch möglich, dass nur ein Verbindungsschlauch vorgesehen ist. Die Schutzelemente 17 können Bestandteil der Haltestruktur sein. Die Schutzelemente 17 sind dann beispielsweise mit den Aufbisskissen 2 und mit dem einen Verbindungsschlauch verbunden. Die Schutzelemente 17 dienen einer zusätzlichen Aussteifung des Mundeinsatzes 16.

Der Mundeinsatz 16 kann eine Kaukraftmesseinheit gemäß der vorherigen Ausführungsform umfassen.

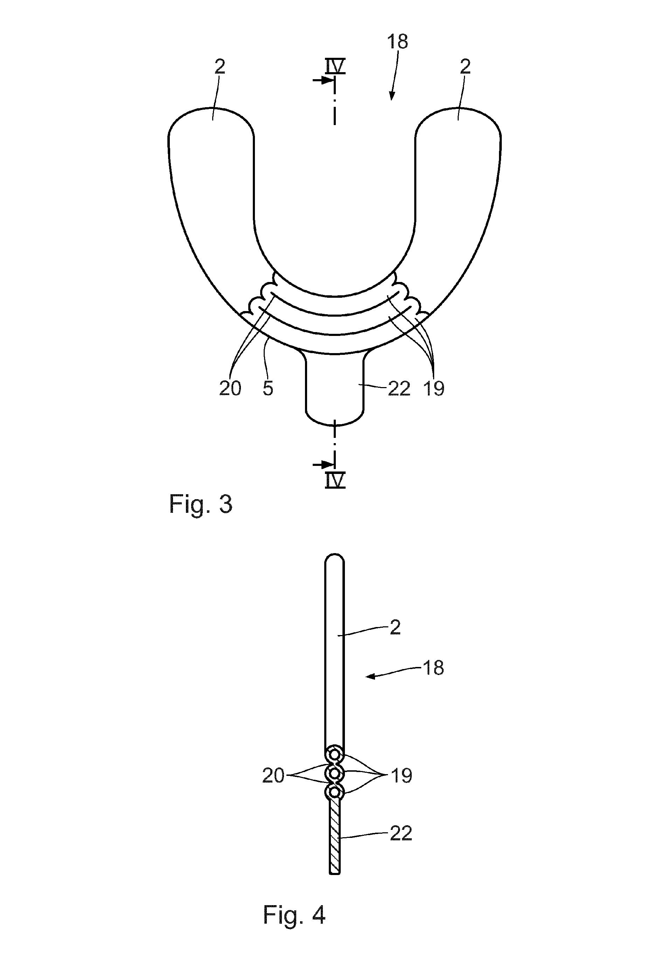

Fig. 3 und 4 zeigen eine weitere Ausführung eines Mundeinsatzes 18. Komponenten, die denjenigen entsprechen, die vorstehend unter Bezugnahme auf die Fig. 1 und 2 bereits erläutert wurden, tragen die gleichen Bezugsziffern und werden nicht nochmals im Einzelnen diskutiert.

Der wesentliche Unterschied gegenüber den beiden ersten Ausführungsbeispielen besteht darin, dass bei dem Mundeinsatz 18 drei Verbindungsschläuche 19 zur Fluidverbindung der Aufbisskissen 2 vorgesehen sind. Der Mundeinsatz 18 weist insgesamt eine flächige Struktur auf. Die Verbindungsschläuche 19 sind in einer Ebene, insbesondere in der Aufbissebene, die von den Aufbisskissen 2 definiert wird, angeordnet. Der Mundeinsatz 18 dient insbesondere zur Verwendung als Beißkissen. Die Verbindungsschläuche 19 sind entlang der gebogenen, U-förmigen Kontur des Mundeinsatzes 18 beabstandet zueinander und parallel zueinander angeordnet. Aufgrund dieser Anordnung ist zwischen jeweils zwei benachbarten Verbindungsschläuchen 19 ein U-förmiger Zwischenraum 20 angeordnet. Ein Patient, der das Beißkissen 18 trägt, kann dieses im Mund intuitiv und unkompliziert mittels der Schneidezähne im Mund positionieren. Dazu ist es lediglich erforderlich, mit den Schneidezähnen in eine der von den Verbindungsschläuchen 19 gebildeten Zwischenräume einzugreifen.

Der Mundeinsatz 18 weist eine im Bereich der Verbindungsschläuche 19 angeordnete Greiflasche 22 auf. Die Greiflasche 22 ermöglicht ein Einsetzen und Herausnehmen des Mundeinsatzes 18. Die Greiflasche 22 ist derart ausgeführt, dass bei einem Tragen des Mundeinsatzes 18 die Greiflasche 22 aus dem Mund des Patienten zwischen den Lippen hervorstehen kann. Die Greiflasche 22 kann ein nicht dargestelltes Filmscharnier aufweisen, um eine Verschwenkbarkeit der, insbesondere außerhalb des Mundes angeordneten, Greiflasche gegenüber den weiteren, insbesondere innerhalb des Mundes angeordneten, Komponenten des Mundeinsatzes 18 zu ermöglichen. Dadurch ist die Handhabung des Mundeinsatzes 18 zusätzlich verbessert.

Der Mundeinsatz 18 weist eine Haltestruktur auf, die parallel zu der Aufbissebene orientiert ist. Die die Aufbisskissen 2 umgebende Hülle 5 ist bei dem Mundeinsatz 18 auch in dem Bereich der Verbindungsschläuche 19 fortgeführt. Das Beißkissen 18 weist eine einheitliche, homogene äußere Struktur auf. Die Verbindungsschläuche 19 sind zwischen den Aufbisskissen 2 in der gemeinsamen Hülle 5 integriert.

Es ist möglich, dass der Mundeinsatz 18 eine Kaukraftmesseinheit gemäß dem ersten Ausführungsbeispiel aufweist.

Der mindestens eine Verbindungsschlauch bildet die Haltestruktur für den sicheren Halt des Mundeinsatzes im Mund. Zusätzliche Haltestrukturen können vermieden werden. Insbesondere ist es nicht erforderlich, zusätzliche Kissen oder andere stabilisierende Maßnahmen, wie Metalldraht-Einlagen, vorzusehen. Der erfindungsgemäße Mundeinsatz ist unkompliziert ausgeführt. Der erfindungsgemäße Mundeinsatz weist eine Eigensteifigkeit und Eigenstabilität auf. Der Mundeinsatz hat eine selbsttragende Struktur. Das bedeutet, dass die Struktur des Mundeinsatzes steif und stabil genug ist, um das Eigengewicht zu tragen. Insbesondere ist die Haltestruktur derartig ausgeführt, dass der Mundeinsatz bei Belastung formstabil ist.

## Patentansprüche

1. Fluidgefüllter Mundeinsatz umfassend
a. zwei Aufbisskissen (2) und
b. mindestens einen die Aufbisskissen (2) verbindenden Verbindungsschlauch (6, 7; 6, 7; 19) zum Austausch von Fluid zwischen den Aufbisskissen (2),
wobei eine die Aufbisskissen (2) verbindende Haltestruktur für einen sicheren Halt des Mundeinsatzes (1; 16; 18) in einem Mund vorgesehen ist,
wobei der mindestens eine Verbindungsschlauch (6, 7; 6, 7; 19) die Haltestruktur bildet.

2. Mundeinsatz gemäß Anspruch 1, **dadurch gekennzeichnet, dass** der Mundeinsatz (1; 16; 18) eine einteilig aus Kunststoff hergestellte Hülle (5) aufweist.

3. Mundeinsatz gemäß einem der vorstehenden Ansprüche, **dadurch gekennzeichnet, dass** genau zwei Verbindungsschläuche (6, 7) zur Ausbildung der Haltestruktur vorgesehen sind, wobei die Haltestruktur quer, insbesondere senkrecht, zu einer von den Aufbisskissen (2) festgelegten Aufbissebene orientiert ist.

4. Mundeinsatz gemäß Anspruch 3, **dadurch gekennzeichnet, dass** ein oberer Verbindungsschlauch (6) oberhalb der Schneidezähne eines Oberkiefers (3) und ein unterer Verbindungsschlauch (7) unterhalb der Schneidezähne eines Unterkiefers (4) anordenbar sind.

5. Mundeinsatz gemäß einem Anspruch 3 oder 4, **dadurch gekennzeichnet, dass** die Verbindungsschläuche (6, 7) jeweils im Bereich der Schlauchmitte ein der Aufbissebene zugewandtes, insbesondere konvex geformtes, Konturelement (9) aufweisen.

6. Mundeinsatz gemäß einem der Ansprüche 3 bis 5, **gekennzeichnet durch** mindestens ein an den Aufbisskissen (2) und/oder den Verbindungsschläuchen (6, 7) angeformtes Schutzelement (17).

7. Mundeinsatz gemäß einem der Ansprüche 1 oder 2, **dadurch gekennzeichnet, dass** mindestens zwei, insbesondere genau drei, Verbindungsschläuche (19) zur Ausbildung der Haltestruktur vorgesehen sind, wobei die Haltestruktur parallel zu einer von den Aufbisskissen (2) festgelegten Aufbissebene orientiert ist.

8. Mundeinsatz gemäß Anspruch 7, **dadurch gekennzeichnet, dass** die Verbindungsschläuche (19) innerhalb der Aufbissebene beabstandet zueinander, insbesondere parallel, angeordnet sind.

9. Mundeinsatz gemäß Anspruch 7 oder 8, **dadurch gekennzeichnet, dass** die Verbindungsschläuche (19) in einer gemeinsamen Hülle (5) integriert sind.

10. Mundeinsatz gemäß einem der vorstehenden Ansprüche, **gekennzeichnet durch** eine Kaukraftmesseinheit umfassend ein 2-Wege-Ventil (12) und einen Druckgeber (13).

11. Mundeinsatz gemäß Anspruch 10, **dadurch gekennzeichnet, dass** das 2-Wege-Ventil (12) und der Druckgeber (13) in einem der Verbindungsschläuche (6, 7; 19) integriert sind.

12. Mundeinsatz gemäß einem der vorstehenden Ansprüche, **dadurch gekennzeichnet, dass** der mindestens eine Verbindungsschlauch (6, 7; 6, 7; 19) aus Ethylenvinylacetat (EVA) oder Silicon hergestellt ist.

13. Mundeinsatz gemäß einem der vorstehenden Ansprüche, **dadurch gekennzeichnet, dass** der mindestens eine Verbindungsschlauch (6, 7; 6, 7; 19) eine Wandstärke im Bereich von 0,5 mm bis 1,0 mm und insbesondere von 0,6 mm bis 0,8 mm und insbesondere von etwa 0,7 mm aufweist.

14. Verwendung eines Mundeinsatzes nach einem der vorstehenden Ansprüche als Sportschiene.

15. Verwendung eines Mundeinsatzes nach einem der Ansprüche 1 bis 13 als Beißkissen.
